# EUROPEAN PATENT SPECIFICATION

(11) **EP 3 723 893 B1**
(45) Date of publication and mention of the grant of the patent: **05.07.2023**
(21) Application number: 18847148.6
(22) Date of filing: 17.12.2018
(51) Int. Cl.: B01D 61/02, B01D 61/14, C08H 1/06, B01D 61/00, C07K 14/47

(54) **METHOD FOR EXTRACTING KERATIN**
VERFAHREN ZUR EXTRAKTION VON KERATIN
PROCÉDÉ D'EXTRACTION DE KÉRATINE

(30) Priority: 15.12.2017 PT 2017110458
(43) Date of publication of application: 21.10.2020
(73) Proprietor: Universidade Católica Portuguesa - UCP, 4169-005 Porto (PT)
(72) Inventor: ESTEVEZ PINTADO, Maria Manuela, 4200-374 Porto (PT); FARIA AMORIM, Maria Manuela, 4200-374 Porto (PT); MENDES FERREIRA MONTEIRO MADUREIRA, Ana Raquel, 4200-374 Porto (PT); ESTEVEZ PINTADO, Ana Isabel, 4200-374 Porto (PT)
(74) Representative: Patentree
(86) International application number: PCT/IB2018/060198
(87) International publication number: WO 2019/116357

(56) References cited:
- WO-A1-2006/042374
- WO-A1-2014/013080
- CN-A- 101 372 503
- US-B2- 8 575 313
- ANA C. CASSONI ET AL: "Novel Eco-Friendly Method to Extract Keratin from Hair", ACS SUSTAINABLE CHEMISTRY & ENGINEERING, vol. 6, no. 9, 6 August 2018 (2018-08-06), pages 12268-12274, XP055570063, US ISSN: 2168-0485, DOI: 10.1021/acssuschemeng.8b02680

## Description

### TECHNICAL FIELD

The present disclosure relates to method for keratin extraction. With this keratin extraction method, it was possible to obtain a keratin product with a protein yield of -50-70%.

This new method uses a single reagent for the digestion of the raw pig hair, followed by mechanical methods, which makes this process simpler, cost-effective and environmentally friendly.

### BACKGROUND

Pig farming is a major worldwide industry that generates many by-products that can cause environmental problems. Nonetheless it is possible to have a management towards the re-use and valorization of these by-products. One opportunity lies in the extraction of keratin present in the pig hair, since it is composed by 80% keratin. There are some methods for keratin extraction from human hair, and other sources as wool and feathers, but they bring many problems as pollution, time-consumption and high costs. This disclosure uses a commercial detergent belonging to the category of degreasers, capable of digesting pig hair easily, followed by a manual filtration to remove unwanted residues from skin trimmings with fat or lard. Then, the resulting solution goes through a process of ultrafiltration to obtain a solution with a higher protein purity.

With this keratin extraction method it was possible to obtain a keratin product with a protein yield of ~50-70%.

Millions of pigs are slaughtered for meat purposes worldwide and are thus the most efficient feed converting animals (Mohan et al., 2014). This industry generates different kinds of by-products that can be lost and cause environmental problems (COWI, 2000). Therefore, management and re-use of these by-products has great importance for a sustainable meat production and is highly suggested.

One example of by-product from this industry is pig hair. The dehairing of pigs consumes a lot of water and generates wastewaters with high levels of organic matter, fat and dirt (COWl, 2000). Hence, the hair is a by-product worthy of management towards its re-use and valorization - nowadays the hair is used for brushes, felt, rugs, upholstery, plaster binding and insulation and glue (COWl, 2000).

One of the major components of hair is keratin, also present in wool, feathers and other keratinous substances, consisting in about 80% keratin (Holkar et al., 2017). There are two major groups: α-keratin, found in the hair, and β-keratin. The α-keratins are sized at 40-65 kDa (Wang et al., 2016) and have a high content of cysteine and stable cross-linked disulfide bridges between different amino acids (Deb-choudhury et al. 2016; Holkar et al. 2017; Langbein et al. 2004; Shavandi et al. 2017). The disulfide bonds provide a compact structure with resistance to chemical or enzymatic reactions (Shavandi et al., 2017) and protective functions to the external environment (Wang et al., 2016). Consequently, keratin is one of the most difficult proteins to digest and extract (Deb-choudhury et al., 2016).

Several studies have reported the extraction of keratin from human hair, however research on the extraction of keratin from the pig hair are scarce. According to a recent review from Holkar et al. (2017), two of the existing methods for keratin extraction from hair are: "Shindai method" (Nakamura et al, 2002) with yield of 75% protein and the "Reduction method" using urea, SDS, and 2-mercaptoethanol with recovery of 78,1 % amino acids.

Document WO2014/013080 discloses a method for extracting keratin from animal hair comprising the steps of: degreasing the animal hair with a 20-80% (V_{degreaser}/Vₜₒₜₐₗ) solution of a degreaser (a non-sulphur containing surfactant) to obtain a solution of degreased animal hair; filtering the solution of degreased animal hair to remove residue and to obtain a solution of keratin.

Nevertheless, various problems are associated with great part of the existing methods such as pollution, high-cost, industry unviability and time-consumption as enumerated by Shavandi et al. (2017), that explores all the methods reported for extracting keratin from various sources.

These facts are disclosed in order to illustrate the technical problem addressed by the present disclosure.

### GENERAL DESCRIPTION

The aim of the present disclosure is to describe an easier method of extracting keratin from pig's hair using a commercial detergent for digestion process followed by ultrafiltration, which makes this method simple with low economic costs and environmentally safer.

The problems above-mentioned are solved by the method disclosed in the present disclosure. This disclosure relates to a method for extracting a non-hydrolyzed α-keratin from animal hair comprising the following steps:
degreasing the animal hair with a 30-60% (V_{degreaser}/Vₜₒₜₐₗ) solution of a degreaser to obtain a solution of degreased animal hair, in particular wherein the step of degreasing is a step of digesting;
filtering the solution of degreased animal hair to remove residues and to obtain a solution of keratin,
   using a sieve with a mesh between 0.5-2 mm; concentrating keratin by ultrafiltration;
   wherein the solution of the degreaser has a pH between 13-14;
   wherein the step of degreasing is carried out for 4-6 hours;
   wherein the obtainable the keratin has a molecular weight between 40-65 kDa.

In a preferred embodiment, the step of degreasing is a step of digesting. This preferred embodiment is combinable with the remaining embodiments of the present disclosure.

In an embodiment, the step of degreasing the animal hair may be carried out with a ratio of 1 kg of animal hair: 4 L of the degreasing solution.

In an embodiment, the step of concentrating keratin is carried out by reverse osmosis or centrifugation or ultrafiltration.

In an embodiment, the step of filtering may be carried out using a sieve with a mesh between 0.5-1 mm.

In an embodiment, the residues may be nails, fat, grease, or combinations thereof.

In an embodiment, the step of degreasing may be carried out at 500-700 rpm.

In an embodiment, the animal hair may be rinsed with water prior to the step of degreasing.

In an embodiment, the animal hair may be a pig's hair.

In an embodiment, the degreaser solution comprises sodium hydroxide, (methyl-2-metoxietoxi)propanol, D-glucopyranose, and N-[3-dimethylamino)propyl], in particular Mistolin^{®}.

In an embodiment, the step of degreasing is carried out at 18-30 °C, preferably at 20-25 °C.

### BRIEF DESCRIPTION OF THE DRAWINGS

The following figures provide preferred embodiments for illustrating the description and should not be seen as limiting the scope of disclosure.

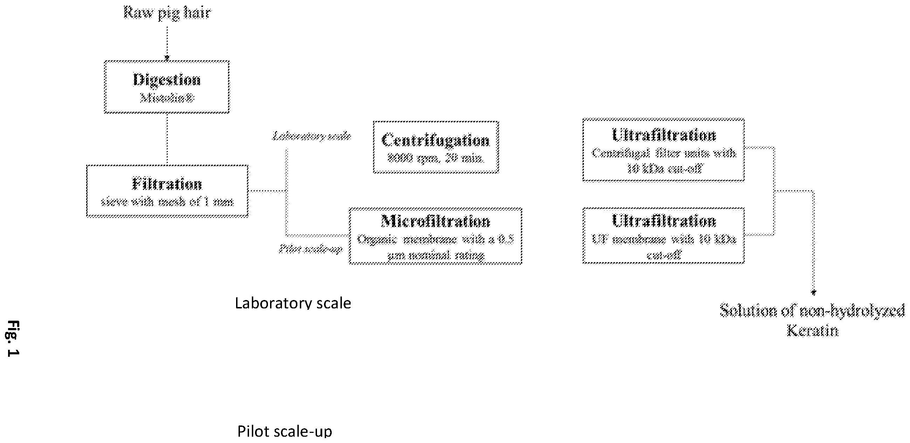

**Figure 1** **-** Schematic representation of the ultrafiltration method (laboratory and pilot scale-up).
**Figure 2** - Aspect of the filtered solution (a) and the residues that are filtrated with the sieve (b).
**Figure 3** - FPLC comparison between the resulting product of the ultrafiltration method (laboratory) (---) and the method described in Gupta et al. 2012 (-).
**Figure 4** - FPLC comparison between the resulting product of the ultrafiltration in laboratory (---) and pilot scale (-).

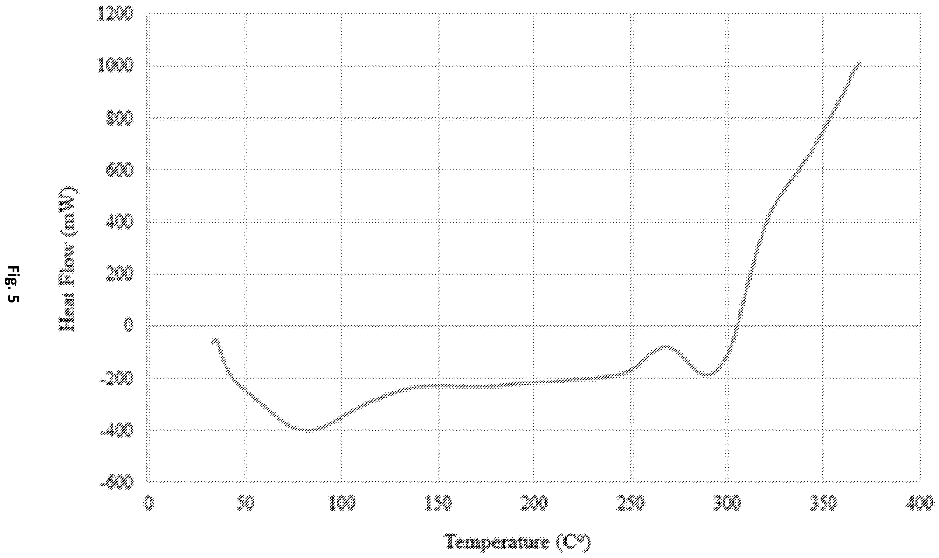

**Figure 5** - DSC curve of the final product of pilot scale extraction heated 10 °C min-1 using aluminum crucibles. Sample mass of ~5 mg.
**Figure 6** - FTIR spectra of the final product of pilot scale extraction.

### DETAILED DESCRIPTION

This disclosure relates to a method for extracting a non-hydrolyzed α-keratin from animal hair, in particular from pigs hair, wherein the method comprises several steps, for example degreasing the animal hair with a solution of a degreaser and filtering the solution of degreased animal hair to remove residues and to obtain a solution of keratin.

The pig hair was provided by ICM Pork, Primor Group (Portugal) as raw material, which brought together residues such as skin with fat, lard, some nails and blood. The raw pig hair was rinsed with tap water to remove bigger residues and then dried and weighed.

In an embodiment, at laboratorial scale, for 100 g of rinsed pig hair, was added 400 mL of Mistolin^{®} HTG 50 (MSTN Group, Portugal) at 50% (v/v), a commercial degreaser, highly alkali (pH=13-14), constituted by nonionic surfactants (<5%) and NaOH. The conditions for the digestion were optimized by trial and error through observation of the digested solution and its precipitation capacity with ammonium sulfate. The digestion/degreasing occurred in particular under the following conditions: 6 hours at room temperature, preferably at 18-30 °C, more preferably 20-25 °C, with stirring at 500-700 rpm in the last 30 min.

In an embodiment, after the pig hair has been digested, the resulting solution was filtered through a sieve having a small mesh (± 1 mm) to remove grease and other solid residues.

In an embodiment, the filtered solution was then centrifuged during 20 min at 8000 rpm. The supernatant was placed in centrifugal filter units (Amicon^{®} Ultra-4, Millipore; Billerica, MA, USA) with a cut-off of 10 kDa and centrifuged during 20 min at 8000 rpm. The supernatant was discarded and the solution above the filter was analyzed.

In an embodiment, for the pilot scale-up tests, 15 Kg of raw pig hair were digested with 60 L of Mistolin^{®} HTG 50 at 50% (v/v) in a large container with continuous stirring of 25 rpm, during 7 hours. Afterwards, the resultant solution was filtered through a sieve with small mesh (± 1mm) to remove fat, nails and other residues. The filtered solution was microfiltered with an ultrafiltration batch equipment (Proquiga, Biotech S.A., La Coruña, Spain) at 40-45 °C using organic membranes MF membrane model FH 3838-LS03-S with a 0,5 µm nominal rating (Iberlact, Madrid, Spain), to remove bigger residues and then passed through a pilot-scale ultrafiltration in the same equipment using UF membrane, model SD 3838 BS03-S with a 10-kDa nominal MWCO (Iberlact, Madrid, Spain). Finally, a reverse osmosis was performed in a pilot plant unit (ORM, Amadora, Portugal) equipped with an OI membrane model 2.5 S Seawater (Advanced Structures Inc., Los Angeles, CA, USA), to concentrate the protein.

Physicochemical analyses were carried out as follows.

In an embodiment, the final solutions of laboratory and pilot scale-up tests were analyzed by Fast Protein Liquid Chromatography (FPLC), Differential Scanning Calorimetry (DSC) and Fourier Transform Infrared Spectroscopy (FTIR).

In an embodiment, in the FPLC, aliquots (100 µL) of ultrafiltrated solution were analyzed by gel filtration chromatography using a Superdex^{™} 200 10/300 GL column connected in series to Superdex Peptide 10/300 GL column (GE Healthcare Life Sciences, Freiburg, Germany), coupled to a FPLC AKTA-purifier system (GE Healthcare Life Sciences, Freiburg, Germany). The eluent used was 0.05 M phosphate buffer pH 7.0, containing 0.15 M sodium chloride (ionic strength) and 0.2 gL-1 of sodium azide (as preservative) at a flow rate of 0.5 mLmin-1. Elution was monitored at 280 nm and approximate molecular mass of protein solutions were determined with standard protein Gel Filtration Calibration Kits (GE Healthcare Life Sciences, Freiburg, Germany) in the range 10 kDa to 669 kDa.

In an embodiment, the total protein content was determined via Kjeldahl method (NP1612, 2006).

In an embodiment, Fourier Transformed InfraRed (FTIR) technique was performed in an ABB MB3000 FT-IR spectrometer (ABB, Zürich, Switzerland) equipped with a horizontal attenuated total reflectance (ATR) sampling accessory (PIKE Technologies, Madison, Wl, USA) with a diamond/ZnSe crystal, obtaining different spectra. The samples were also precipitated with ammonium sulfate since the concentration of the liquid product wasn't sufficient.

In an embodiment, both FPLC and FTIR analysis occurred in triplicate and were compared to samples obtained from Gupta et al. 2012.

In an embodiment, the Differential Scanning Calorimetry was performed in DSC-60 (Shimadzu, Columbia, USA) using ~5 mg of sample obtained from pilot scale-up extraction, in triplicate, and placed in an aluminum crucible. The samples were previously precipitated with ammonium sulfate since the system requires dry samples. The thermal behavior was evaluated in the temperature range of 30-350 °C at a heating rate of 10 °C/min. Enthalpy values and optimal melting temperatures were calculated by the equipment software (ta60 version 2.10, DSC software, Shimadzu, Columbia, USA).

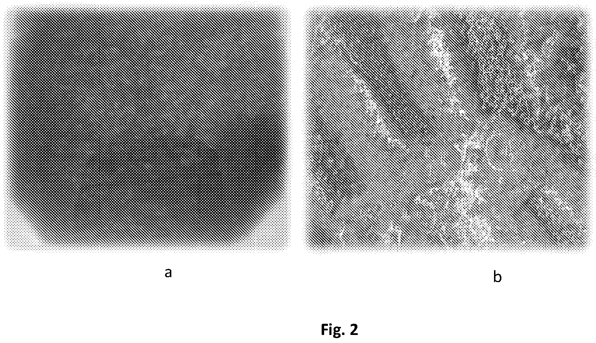

In an embodiment, the visual aspect of the digested material was carried out. In the solution resulting from the digestion with the commercial solvent Mistolin^{®}, no hair fibers were observed, it being concluded that the digestion was practically complete. Subsequent filtration has also been able to remove residues of fat and other solid residues still remaining in the digested solution which have an appearance as a slightly pasty solution of light brown color (Fig. 2b). The resulting filtration solution showed a brown color with small residues in suspension (Fig. 2a).

In an embodiment, the determination of the total protein content (Table 1) shows that the performance of ultrafiltration resulted in the gathering of samples with comparable results to the ones obtained by the method described in Gupta et al. (2012) at a laboratory scale. At a pilot scale, the lower protein content can be due to a purer extract, i.e. the final product consists in an extract of keratin with little residues comparing with the other methods that may have other proteins.

**Table 1 - Total protein content of the different processes (ultrafiltration, laboratory and scale-up and precipitation method, as described in Gupta et al. 2012) determined by Kjeldhal method.**

| Process | Total protein content (%) |
|---|---|
| Ultrafiltration - Laboratory | 61.93 (± 4.41) |
| Ultrafiltration - Scale-up | 36.27 (± 9.31) |
| Precipitation & recovery - (Gupta et al., 2012) | 82.16 (± 2.45) |

In an embodiment, in terms of protein yield, the Shindai method (Nakamura et al., 2002) reports a protein yield of 78% for rat hair extraction, in a laboratory scale. Moreover, the study of Gupta et al. (2012), reports a 53% protein yield in the extraction of keratin from chicken feathers. Hence, the ultrafiltration method is adequate in terms of recovery of protein, comparable to other methods having a satisfactory protein yield (Table 2). In addition, the pilot scale ultrafiltration can maintain a reasonable protein yield.

**Table 2 - Protein yield (%) of the different processes (ultrafiltration, laboratory and scale-up, and precipitation method as described in Gupta et al. 2012).**

| Process | Protein yield (%) |
|---|---|
| Ultrafiltration (laboratory) | 70 (± 7.8) |
| Ultrafiltration (scale-up) | 53.5 (± 4.9) |
| Precipitation & recovery (Gupta et al., 2012) | 73.3 (± 3.5) |

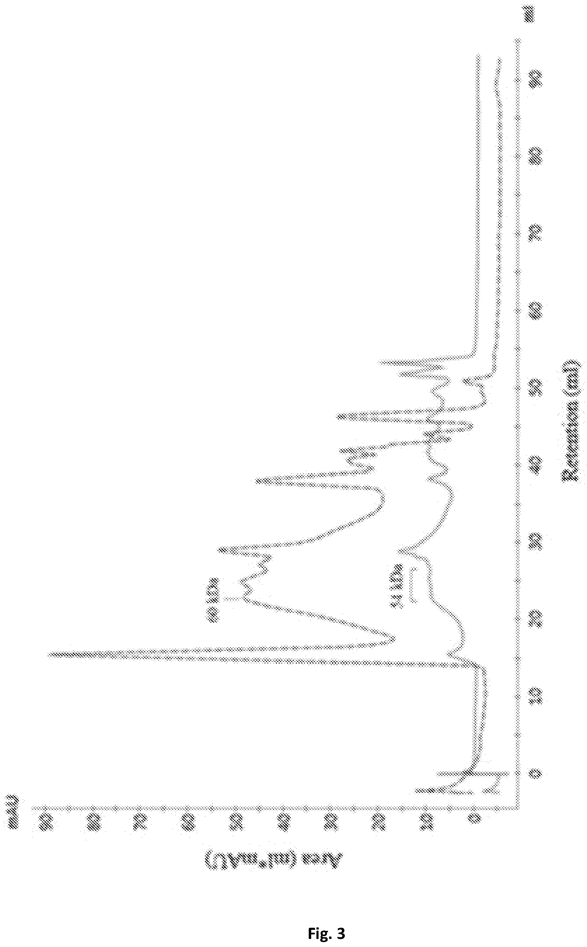

In an embodiment, in the FLPC profile was possible to identify different peaks that represent different molecular weights, and through the determination of the molecular weights it was possible to associate to the molecular weights of keratin. Keratin present in the pig hair (a-keratin) has a molecular weight between 40-65 kDa (Wang et al., 2016). The solutions analyzed by FPLC showed that for the protein sample obtained with laboratory ultrafiltration method (Fig. 3), a peak corresponding to 61 kDa, and for the samples obtained by the method described in Gupta et al. (2012) (Fig. 3) a peak of 54 kDa. Both peaks are within the molecular weight range of keratin.

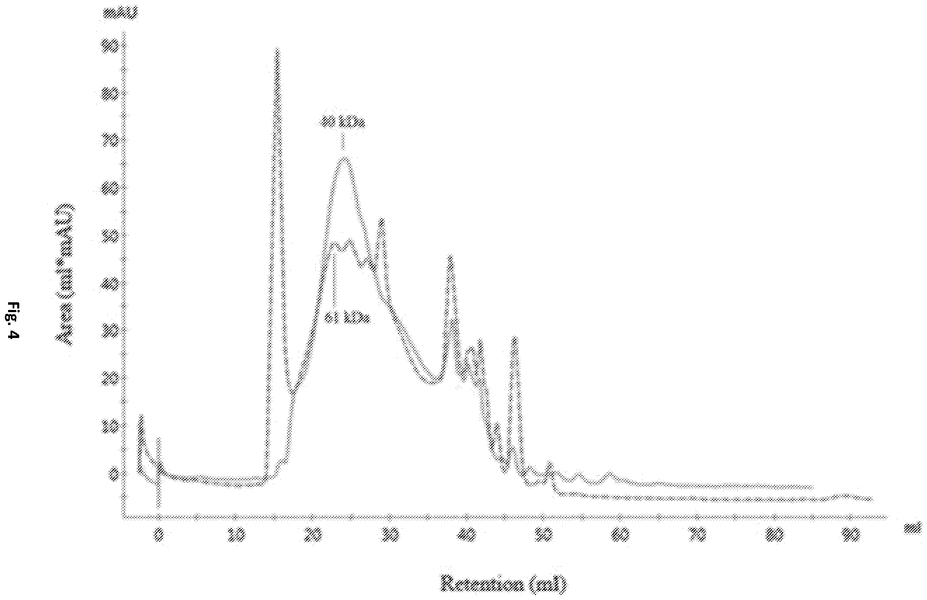

In an embodiment, comparing the proteins obtained by both ultrafiltration methods (Fig. 4) performed at lab and pilot scale showed peaks that correspond to the molecular weight of keratin. The pilot scale product has a more defined and concentrated peak which also shows a purer final product.

In an embodiment, the DSC curve shows an initial endotherm peak around 70 °C due to water loss of the sample (Popescu & Gummer, 2016). At 280 °C it is possible to observe another peak that may be due to the denaturing of keratin as reported by other studies (Dankers, 2007; Popescu & Gummer, 2016). This result reinforces that the final product obtained by ultrafiltration method is keratin.

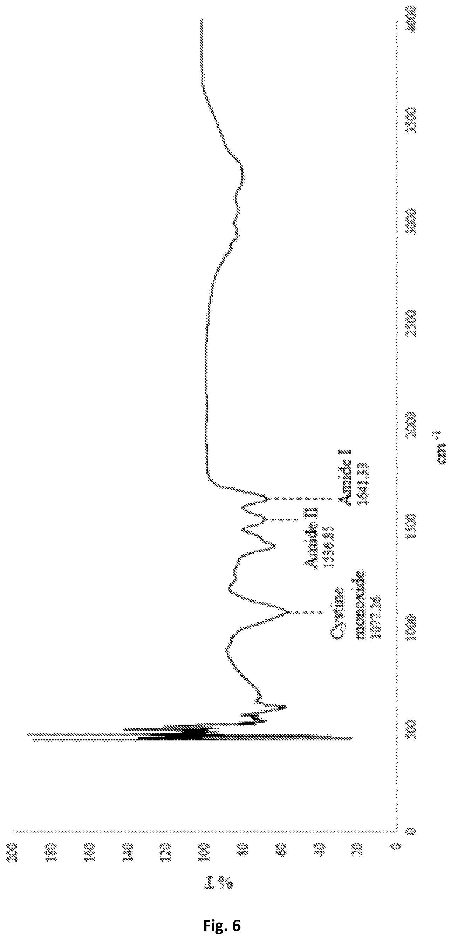

In an embodiment, it is possible to identify the amide and cystine peaks in the FTIR spectra (Fig. 6), which proves the existence of true protein in the sample. Also, this result is comparable to Dankers, 2007 where various samples of hair were analyzed.

In an embodiment, reported studies use different reagents to perform the extraction of keratin, which are harmful to the environment. As example, the method described by Gupta et al. (2012) uses ether, sodium sulphide and ammonium sulfate, and the Shindai method uses ethanol, chloroform, methanol, Tris-HCL, thiourea, urea and 2-mercaptoethanol. The method described in this disclosure uses only Mistolin^{®} as solvent reagent and ultrafiltration which is environmental friendly, not only because of the reduction of the reagents used but also because Mistolin^{®} is a biodegradable detergent used by industry. However, this reagent is highly alkaline due to the presence of NaOH. Regarding to the execution time, this method takes about 9 h to get the final product, which is much lower than the Shindai and Gupta methods that take about 2 days. The final product obtained is a brown liquid with a pH of 12.5 and a fairly noticeable smell.

In an embodiment, pig hair is a by-product of the pig farming industry that has a high percentage of keratin and since the dehairing can potentially cause environmental problems, its re-use is of highly importance. In this research it was developed a method to extract keratin from pig hair that uses only one reagent and is mostly mechanical - ultrafiltration method. The yield of protein is similar to other studies reported and when compared, it was possible to significantly reduce the amount of time spent. The pilot scale-up was proved to be effective, thus this method has the potential to be applied in an industrial level. The final product consists in a fairly pure extract keratin, as show in FPLC and DSC. Also, as the method uses a commercial degreaser, we believe that it can be performed in other keratinous by-products.

The keratin extraction method of the present disclosure is innovative, simpler, cost-effective and environmental friendly.

In an embodiment, was measured differences and advantages between the works:
- Eco-friendly method to extract keratin from hair of the (A),
- Comparison of methods for extraction of Keratin from Waste Wool (B).

The methods above mentioned aim to extract keratin from meat industries residues. The method proposed to patent (A) refers to keratin extracted from pig hair and the comparison method (B) uses wool waste to extract keratin, therefore the residues used are different being the pig hair a residue never used for this purpose.

In an embodiment, the work developed for keratin extraction from wool waste starts with a step of removal of residual lipid that uses various potentially harmful reagents such TWEEN, chloroform, methanol or petroleum ether and with an execution time of from 2, 6 or 24 hours. The extraction method for pig hair does not require previous lipid removal, the hair is only rinsed to remove residues of larger dimension.

The solubilization of the wool waste was tested with various published methods (table 1) that also used various reagents and with a long execution time. Regarding the novel method, it was used only commercial reagent (Mistolin^{®}) for the solubilization of pig hair followed by a filtration with a sieve and an ultrafiltration to purify protein. Also, it is possible to perform a reverse osmosis to concentrate protein. With the reduction of reagents used and time spent in this method, it is possible to reduce significantly the costs of extraction.

**Table 3 - Reagents used and time spent in the solubilization of wool waste and pig hair.**

| | **Solubilization method** | **Reagents** | **Time (hours)** |
|---|---|---|---|
| **Wool waste** | **Oxidation-Peracetic Acid (PAA)** | Peracetic acid | 27 |
| | | Tris | |
| | | HCl | |
| | **Oxidation-Percarbonate (PCC)** | NaOH | 16 |
| | | Sodium percarbonate | |
| | **Reduction/Oxidation-Thioglycolate/Hydrogen Peroxide (TGA)** | Sodium thioglycolate | 27 |
| | | HCl | |
| | | H2O2 | |
| | **Denaturation/Reduction-Urea/2-Mercaptoethanol (UTM)** | Tris | 72 |
| | | Thiourea | |
| | | Urea | |
| | | 2-mercaptoethanol | |
| | **Denaturation/Reduction-Urea/ Metabisulfite (SMB)** | Urea | 5 |
| | | SDS | |
| | | Sodum metabisulfide | |
| | **Reduction-Sulfide (SUL)** | Sodium sulfide | 48 |
| | | Calcium oxide | |
| **Pig hair** | **Method of the present disclosure** | Mistolin^{®} | 7 |

The method for extracting keratin of the present disclosure is innovative, simpler, quick, cost-effective and environmental friendly.

The term "comprising" whenever used in this document is intended to indicate the presence of stated features, integers, steps, components, but not to preclude the presence or addition of one or more other features, integers, steps, components or groups thereof.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

### References

- COWI Consulting Engineers and Planners AS. (n.d.). Cleaner Production Assessment in Meat Processing.
- CT 35. (2006). NP 1612 - Carnes e produtos cárneos; Determina 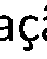 ão do teor de azoto total (Método de referência).
- Dankers, L. M. (2007). Physical analysis of human hair. Missouri University of Science and Technology.
- Deb-choudhury, S., Plowman, J. E., & Harland, D. P. (2016). Isolation and Analysis of Keratins and Keratin-Associated Proteins from Hair and Wool. Intermediate Filament Proteins (1st ed., Vol. 568). Elsevier Inc. http://doi.org/10.1016/bs.mie.2015.07.018
- Gupta, A., Binti, N., Universiti, K., Kee, G., Universiti, C., Introducing, A., ... Gupta, A. (2012). Extraction of Keratin Protein from Chicken Feather. Journal of Chemistry and Chemical Engineering, (August), 732-737.
- Holkar, C. R., Jain, S. S., Jadhav, A. J., & Pinjari, D. V. (2017). Valorization of keratin based waste. Process Safety and Environmental Protection, 1-14. http://doi.org/10.1016/j.psep.2017.08.045
- Langbein, L., Spring, H., & Rogers, M. A. (2004). Hair Keratins and Hair Follicle - Specific Epithelial Keratins. Methods in Cell Biology, 78, 413-451.
- Mohan, N. H., Debnath, S., Mahapatra, R. K., Nayak, L. K., Baruah, S., Das, A., ... Tamuli, M. K. (2014). Tensile properties of hair fibres obtained from different breeds of pigs. Biosystems Engineering, 119, 35-43. http://doi.org/10.1016/j.biosystemseng.2014.01.003
- Nakamura, A. N., Arimoto, M., Takeuchi, K. T., & FUjii, T. F. (2002). A Rapid Extraction Procedure of Human Hair Proteins and Identification of Phosphorylated Species, 25(May), 569-572.
- Popescu, C., & Gummer, C. (2016). DSC of Human Hair : A tool for claim support or incorrect data analysis? Internatioal Journal of Cosmetic Science, (January), 1-7. http://doi.org/10.1111/ics.12306
- Shavandi, A., Silva, T. H., Bekhit, A. A., & Bekhit, A. E.-D. A. (2017). Keratin: dissolution, extraction and biomedical application. Biomaterials Science, 5, 1699-1735. http://doi.org/10.1039/c7bm00411g
- Wang, B., Yang, W., Mckittrick, J., & Meyers, M. A. (2016). Keratin : Structure , mechanical properties , occurrence in biological organisms , and efforts at bioinspiration. JOURNAL OF PROGRESS IN MATERIALS SCIENCE, 76, 229-318. http://doi.org/10.1016/j.pmatsci.2015.06.001

## Claims

1. Method for extracting a non-hydrolyzed α-keratin from animal hair comprising the following steps:
degreasing the animal hair with a 30-60% (v_{degreaser}/vₜₒₜₐₗ) solution of a degreaser to obtain a solution of degreased animal hair, wherein the step of degreasing is a step of digesting;
filtering the solution of degreased animal hair to remove residues and to obtain a solution of keratin using a sieve with a mesh between 0.5-2 mm; concentrating keratin by ultrafiltration;
wherein the solution of the degreaser has a pH between 13-14;
wherein the step of degreasing is carried out for 4-6 hours;
wherein the obtainable keratin has a molecular weight between 40-65 kDa.

2. Method according to the previous claim, wherein the step of degreasing the animal hair is carried out with a solution of 50% (v_{degreaser}/vₜₒₜₐₗ).

3. Method according to any of the previous claims, wherein the step of degreasing the animal hair is carried out with a ratio of 1 kg of animal hair: 4 L of the degreasing solution.

4. Method according to any of the previous claims, further comprising a step of concentrating the solution of degreased animal hair to obtain a concentrated solution of keratin.

5. Method according to any of the previous claims, wherein the step of filtering is carried out using a sieve with a mesh between 0.5-1 mm.

6. Method according to any of the previous claims, wherein the residues are nails, fat, grease, or combinations thereof.

7. Method according to any of the previous claims, wherein the step of degreasing is carried out at 500-700 rpm.

8. Method according to any of the previous claims, wherein the hair is rinsed with water prior to the step of degreasing.

9. Method according to any of the previous claims, wherein the animal hair is pig's hair.

## Patentansprüche

1. Verfahren zur Extraktion eines nicht-hydrolysierten α-Keratins aus Tierhaar, umfassend die folgenden Schritte:
Entfetten des Tierhaars mit einer 30-60%igen (v_{Entfetter}/v_{gesamt}) Lösung eines Entfetters, um eine Lösung aus entfettetem Tierhaar zu erhalten, wobei der Schritt des Entfettens ein Schritt des Digerierens ist;
Filtrieren der Lösung aus entfettetem Tierhaar, um Rückstände zu entfernen und eine Kreatinlösung zu erhalten, unter Verwendung eines Siebs mit einer Maschenweite zwischen 0,5-2 mm; Konzentrieren des Keratins durch Ultrafiltration;
wobei die Lösung des Entfetters einen pH-Wert zwischen 13-14 aufweist;
wobei der Schritt des Entfettens 4-6 Stunden lang erfolgt;
wobei das erreichbare Keratin ein Molekulargewicht zwischen 40-65 kDa aufweist.

2. Verfahren nach dem vorangehenden Anspruch, wobei der Schritt des Entfettens des Tierhaars mit einer Lösung von 50 % (v_{Entfetter}/v_{gesamt}) erfolgt.

3. Verfahren nach einem der vorangehenden Ansprüche, wobei der Schritt des Entfettens des Tierhaars in einem Verhältnis von 1 kg Tierhaar zu 4 L Entfettungslösung erfolgt.

4. Verfahren nach einem der vorangehenden Ansprüche, ferner umfassend einen Schritt des Konzentrierens der Lösung aus entfettetem Tierhaar, um eine konzentrierte Keratinlösung zu erhalten.

5. Verfahren nach einem der vorangehenden Ansprüche, wobei der Schritt des Filterns unter Verwendung eines Siebes mit einer Maschenweite zwischen 0,5-1 mm erfolgt.

6. Verfahren nach einem der vorangehenden Ansprüche, wobei die Rückstände Nägel, Fette, Schmiermittel, oder Kombinationen davon sind.

7. Verfahren nach einem der vorangehenden Ansprüche, wobei das Entfetten bei 500-700 U/min erfolgt.

8. Verfahren nach einem der vorangehenden Ansprüche, wobei das Haar vor dem Schritt des Entfettens mit Wasser ausgespült wird.

9. Verfahren nach einem der vorangehenden Ansprüche, wobei das Tierhaar Schweinehaar ist.

## Revendications

1. Procédé d'extraction d'une kératine α non hydrolysée à partir de poil d'animal comprenant les étapes suivantes :
dégraisser le poil d'animal avec une solution 30-60% (V_{dégraissant}/Vₜₒₜₐₗ) d'un dégraissant pour obtenir une solution de poil d'animal dégraissé, dans lequel l'étape de dégraisser est une étape de digérer ;
filtrer la solution de poil d'animal dégraissé pour éliminer les résidus et pour obtenir une solution de kératine à l'aide d'un tamis avec un maillage entre 0,5-2mm ; concentrer la kératine par ultrafiltration ;
dans lequel la solution du dégraissant a un pH situé entre 13-14 ;
dans lequel l'étape de dégraisser est exécutée durant 4-6 heures ;
dans lequel la kératine pouvant être obtenue a un poids moléculaire situé entre 40-65 kDa.

2. Procédé selon la revendication précédente, dans lequel l'étape de dégraisser le poil d'animal est exécutée avec une solution de 50% (V_{dégraissant}/Vₜₒₜₐₗ).

3. Procédé selon l'une quelconque des revendications précédentes, dans lequel l'étape de dégraisser le poil d'animal est exécutée avec un ratio de 1 kg de poil d'animal : 4L de solution dégraissante.

4. Procédé selon l'une quelconque des revendications précédentes, comprenant également une étape de concentrer la solution de poil d'animal dégraissé pour obtenir une solution concentrée de kératine.

5. Procédé selon l'une quelconque des revendications précédentes, dans lequel l'étape de filtrer est exécutée à l'aide d'un tamis avec un maillage entre 0,5-1mm.

6. Procédé selon l'une quelconque des revendications précédentes, dans lequel les résidus sont des ongles, de la matière grasse, de la graisse ou des combinaisons de ceux-ci.

7. Procédé selon l'une quelconque des revendications précédentes, dans lequel l'étape de dégraisser est exécutée à 500-700 rpm.

8. Procédé selon l'une quelconque des revendications précédentes, dans lequel le poil est rincé avec de l'eau avant l'étape de dégraissage.

9. Procédé selon l'une quelconque des revendications précédentes, dans lequel le poil d'animal est du poil de cochon.
